# NEW EUROPEAN PATENT SPECIFICATION

(11) **EP 2 763 706 B2**
(45) Date of publication and mention of the opposition decision: **26.10.2022**
(45) Mention of the grant of the patent: 17.07.2019
(21) Application number: 12781050.5
(22) Date of filing: 04.10.2012
(51) Int. Cl.: A61L 9/14, A61L 9/12, A61L 9/03

(54) **AIR FRESHENING NETWORK**
NETZWERK FÜR LUFTERFRISCHUNG
RÉSEAU DE RAFRAÎCHISSEMENT D'AIR

(30) Priority: 03.10.2011 US 201161542312 P; 24.08.2012 US 201213593769
(43) Date of publication of application: 13.08.2014
(73) Proprietor: The Procter & Gamble Company, Cincinnati, OH 45202 (US)
(72) Inventor: HASENOEHRL, Erik, John, Loveland, OH 45140 (US); GRUENBACHER, Dana, Paul, Fairfield, OH 45014 (US)
(74) Representative: P&G Patent Belgium UK
(86) International application number: PCT/EP2012/069673
(87) International publication number: WO 2013/050506

(56) References cited:
- WO-A1-2004/043502
- GB-A- 2 233 230
- US-A1- 2004 028 551
- US-A1- 2005 129 568
- US-A1- 2007 109 763
- US-A1- 2008 265 799
- US-A1- 2010 044 453

## Description

### FIELD OF THE INVENTION

The present invention is directed to providing air freshening in a manner that is energy efficient and intelligent. In particular, the present invention uses distributed processing across a network of air fresheners to control the odor level for a given environment.

### BACKGROUND OF THE INVENTION

Devices that contain both a sensor and scent emitter have been described. For example if the sensor is an odor sensor, and the odor senses a malodor, the sensor will cause the scent emitter to emit a diffusible air freshening composition into the air to mask and/or eliminate the offending malodor. However, these devices typically work as a stand-alone device that can only affect a relatively small environment that is immediately surrounding the device and without regard to other air fresheners that may be elsewhere in the home or office. Furthermore, the sensor and the scent emitter are integrated into a single device (i.e., the scent emitter and sensor are not remote to each other).

Many homes or offices (i.e., macro environments) typically have multiple rooms or micro environments that cannot be readily addressed by a single scent emitting device. Often, there is an unbalance in scent level as the user travels from one space to another. The use of a plurality of scent emitting devices fails to provide an optimized air freshening experience throughout the macro environment. For example, a small room may have an over abundance of scent where a larger room may have a scent level that is too low. Some devices will have a manual scent intensity control but the user's preferred level is typically obtained by trial and error. Or the manual scent intensity control simply lacks precision. Further, a collection of single isolated devices often fail to anticipate the effect of a malodor event that may happen at a single location in the home or office that leads to an undesirable olfactory experience elsewhere in the macro environment. For example, frying a fish in a kitchen may lead to malodor not only in the kitchen but also in adjoining rooms (as the malodor diffuses). In such an example, there is a need to proactively address the malodor event elsewhere in the home besides the kitchen. Current devices fail to address such an event.

There is a need for a system of devices that addresses the challenges of micro environments in a home or office that cannot be addressed by a single air freshening device. A plurality of devices can be utilized to address each micro environment but such an approach fails to leverage the potential advantages of a system, i.e., the devices working in concert together. There is a need to detect odors in a home or office and provide a user's desired air freshening experience throughout a macro environment. There is a need to provide this experience according to a user's preference, that is simple to operate, and optimizes energy consumption and/or consumables (e.g., scent refill).

### SUMMARY OF THE INVENTION

The present invention attempts to meet these and other needs by providing an air freshening system comprising: (a) a plurality of scent emitters displaced relative to each other in the freshening system, wherein each of the plurality of scent emitters is configured to emit an air freshening composition; (b) at least one sensor responsive to a stimulus the sensor is capable of sensing; (c) a communication interface configured to communicate with the plurality of scent emitters; (d) a director; and (e) wherein each of the plurality of scent emitters comprises an emission processor and the director is configured to deliver a user's requests to the emission processors, wherein the emission processor is: responsive to the sensor; configured to controlling scent emission from the respective scent emitter; and configured to make scent emission decisions through distributed processing across the system based on information exchange among the scent emitters through the communication interface; and configured to learn behavior desired by the user by responding to the user's requests and modifying its behavior in response to the user's requests.

Another aspect of the invention provides a scent emitter configured to emit an air freshening composition comprising: an energizable dispenser component configured to dispense an air freshening composition from a refill vial; a communication interface; an emission processor, wherein the emission processor is configured: to control scent emission for the scent emitter; and to make scent emission decisions based on information received wirelessly.

Yet another aspect of the invention provides for a system comprising an air filtering device configured to filter air comprising: (a) an air filtering component configured to move air through an air filter; (b) optionally having the air filter functionally attached; (c) a communication interface; and (d) an air filtering processor, wherein the air filtering processor is configured: to control air flow through the air filter; and to make air filtering decisions based on information received wirelessly by the communication interface.

Other aspects of the invention provide for methods of eliminating odor or freshening the air comprising the use of the systems and products described herein. Another method is directed to improving air quality in a home or office by the use of the systems or products described herein.

### BRIEF DESCRIPTION OF THE DRAWINGS

FIG. 1 shows the system of architecture of a basic Scent Emission Control Network (SECN) application.
FIG. 2 shows additional system components that can be integrated with the basic application shown in FIG. 1.
FIG. 3 shows applicability of SECN control techniques to other types of device.
FIG. 4 shows the elements of an SECN controller.
FIG. 5 shows components internal to a full-function director.
FIG. 6 shows the structure of software components in a typical controller implementation.

### DETAILED DESCRIPTION OF THE INVENTION

The present invention integrates a control microprocessor with each scent emitter and/or sensors and enabling the control microprocessors to communicate with each other to provide coordinated behaviors across a collection of scent emitters. Such coordination is particularly valuable for scent emitters within a single macro environment (e.g., home or office), where it is desirable for multiple scent emitters (and optionally multiple sensors) to operate together to provide odor control and/or air freshening that is perceived by users as being uniform and effective. Still further, such a system would allow for coordinated responses not only to a malodor event but also to mitigate against scent habituation. Approaches to mitigate scent habituation include turning scent emitters "off' for a period of time (before turning back on again) and/or alternating scents. Although these approaches may have been suggested for single devices, a system approach as described by the present invention provides an advantage by coordinating the behavior of scent emitters across a macro environment. For example, a first scent emitter can be turned off while a second scent emitter can be turned on. Of course there could be overlap between the emitters, and different or complementary scents can be used. All the scent emitters could be turned "off' at night or when occupants are away or asleep to conserve power and/or air freshening composition.

Integrating a control microprocessor with each scent emitter and/or sensor allows the controlled units to be programmed with different behaviors. Behaviors (for example, the ability to learn and replay scent emission patterns) can be delivered to the control microprocessors as independent software modules. Such modules could, for example, be provided as separately purchased software upgrades for existing hardware, enabling scent emitters to provide more sophisticated functions with no change, modification, or alteration to the sources themselves.

In one aspect, the present invention describes an architecture for controlling the operation of scent emitters and/or sensors. The architecture provides for self-organizing autonomous control: a system in which elements such as scent emitters/ sensors communicate and interact with each other to provide behaviors appropriate to the environment in which they operate, based on minimal human interaction and configuration. The system learns the desired behavior by responding to human requests and modifying its behavior in response to those requests. One aspect of the invention is that behaviors can be defined by independently loadable software modules that are installed on a within the system elements so that an individual element can exhibit a wide variety of behaviors.

One goal of the Scent Emission Control Network (SECN) architecture is to combine the many advantages multiple scent emitters in a macro environment (and optionally one or more sensors) with the additional capabilities provided by integrating local digital microprocessor control into each scent emitter. The combination of these scent emitters and/or sensor(s) with autonomous distributed control can further reduce energy costs and/or perfume composition refills by ensuring that perfume compositions are emitted only when actually needed. In addition, once such a control mechanism is present, the same control, sensor, and communication facilities can enable a wide variety of other functions for behavior customization, system control, and integration with building management. In addition, since the control platform is built around a general-purpose microprocessor running an arbitrary set of software modules, the system's control, sensor, and communication functions of the system can also be used to control arbitrary other types of devices, and to provide transport for other types of data. In one embodiment, the system of the present invention comprises non-scent emitting devices. Non-limiting examples include light emitting devices or air filtration devices. See e.g., US 8,035,320.

### Architectural Components

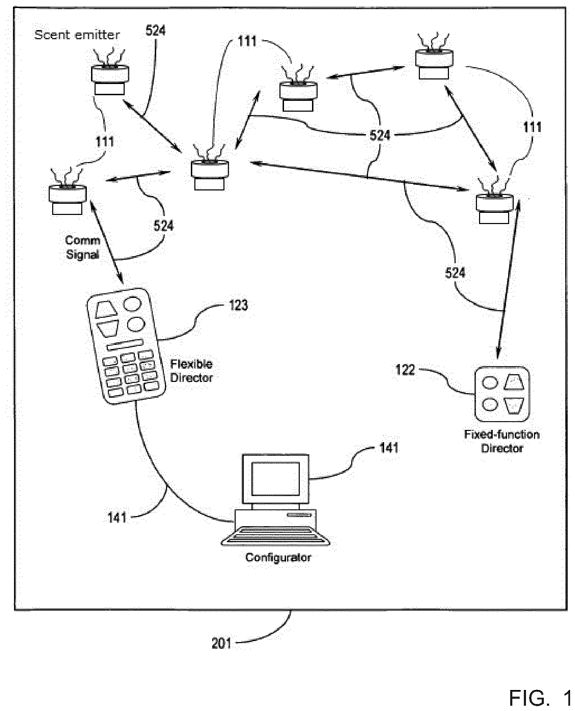

FIG. 1 shows an example SECN implementation comprising plural scent emitters 111, a fixed-function director 122, a flexible director 123, and a configurator 141. All these elements are installed and/or operated within a home or office 201. These elements communicate with each other by sending messages with infrared communication signals 524.

There are many possible designs for scent emitter 111, depending on the amount and/or speed of scent emission, power sources (e.g., AC line, DC, battery), malodor considerations, and control requirements. A non-limiting example includes pluggable devices sold under the FEBREZE brand (manufactured by The Procter & Gamble Company). One such device is FEBREZE NOTICEABLES.

In one embodiment, the scent emitter comprises an energizable dispenser component configured to dispense an air freshening composition from a refill vial. The vial is releasably attached to the dispenser component. The dispensing component may comprise a wick heater or vibrator (e.g., piezoelectric). "Energizable" as used herein refers to a system that operates by using an electrical energy source to emit a targeted active. One example of an energized vapor phase system is a liquid electric air freshening device. Non-limiting examples of an energized system include a wick system (preferably heating the wick or composition per U.S. US 7,223,361), vibration (e.g., ultrasonic or piezoelectric per US 2011/0266359 A1), or combinations thereof.

The air freshening composition may be contained in a vial for dispensing. A non-limiting example of a vial includes a scented oil refills for FEBREZE NOTICEABLES (P&G). In one embodiment, the vial contains from about 5 ml to about 250 ml, alternatively from 25 ml to about 125 ml, alternatively from about 50 ml to about 150 ml, alternatively combinations thereof. The vial may be plastic or glass or combination thereof. The vial may be a consumable, i.e., replaceable by the user as it becomes depleted.

The air freshening composition may include perfume ingredients to provide a desirable scent in the air. In one embodiment, the air freshening composition includes a mixture of volatile aldehydes that are designed to deliver genuine malodor neutralization (and not function merely by covering up or masking odors). A genuine malodor neutralization provides a sensory and analytically measurable (e.g. gas chromatograph) malodor reduction. Thus, if the air freshening composition delivers genuine malodor neutralization, the composition will reduce malodors in the vapor and/or liquid phase. In another embodiment, the air freshening composition comprises a mixture of volatile aldehydes that neutralize malodors in vapor and/or liquid phase via chemical reactions. Such volatile aldehydes are also called reactive aldehydes. Volatile aldehydes may react with amine-based odors, following the path of Schiff-base formation. Volatiles aldehydes may also react with sulfur-based odors, forming thiol acetals, hemi thiolacetals, and thiol esters in vapor and/or liquid phase. It may be desirable for these vapor and/or liquid phase volatile aldehydes to have virtually no negative impact on the desired perfume character of the air freshening composition. In one embodiment, the sensor (e.g., odor sensor) is integrated as part of the scent emitter device. See e.g., US 2010/0044453 A1.

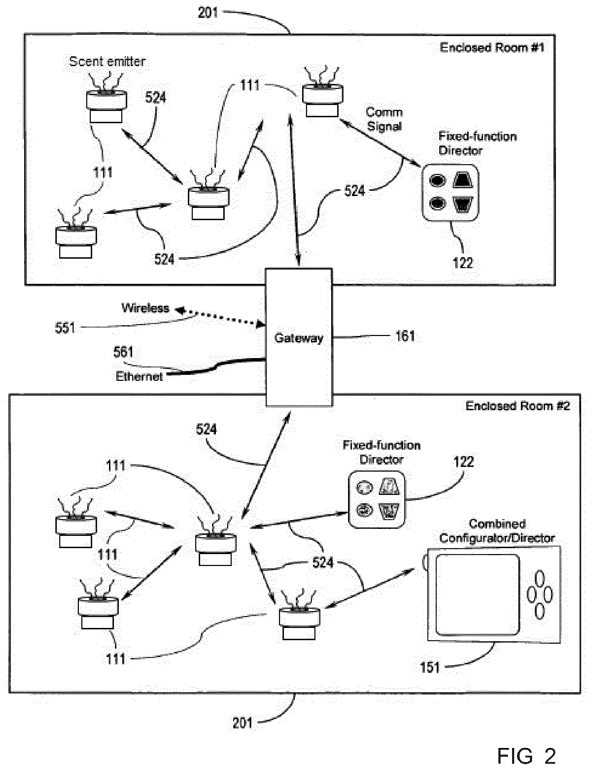

FIG. 2 shows a more complex example SECN implementation consisting of plural rooms 201 in a macro environment (e.g., home or office), optically isolated from each other by opaque room walls, each containing plural scent emitters 111. In FIG. 2, combined configurator/director 151 takes the place of configurator 141 and flexible director 123. Fixed-function directors 122 are present in each of rooms 201. Gateway 161 enables communication between different rooms 201, which would otherwise block infrared control messages 524 with their opaque walls. Gateway 161 can also incorporate other communication interfaces, such as wireless signal 551 or Ethernet interface 561.

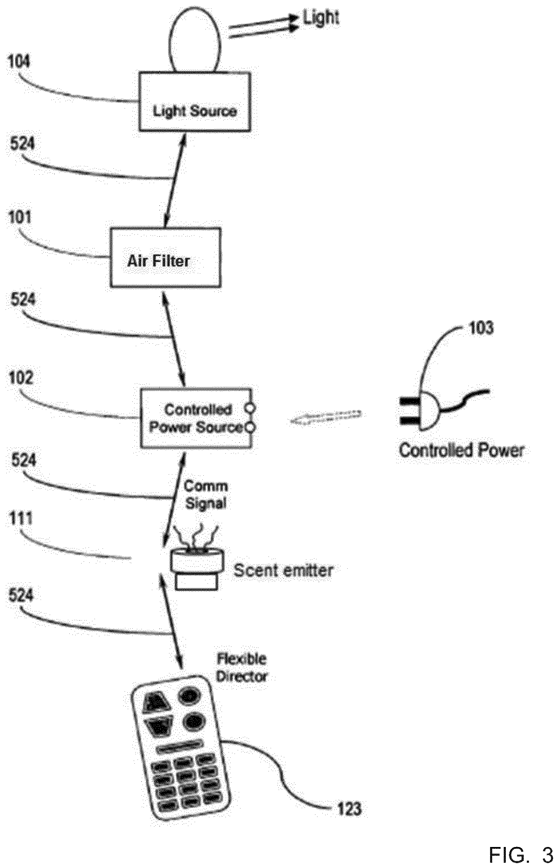

As is evident from the description herein, FIGS. 1 and 2 are only examples of potentially arbitrary combinations of elements in a SECN implementation. Because scent emitters 111 provide an easily-understood target for SECN control capabilities, they are used in most examples here. However, scent emitter 111 is simply one type of appliance 101 that can be controlled with SECN control capabilities. As shown in FIG. 3, flexible director 123 can be used to control arbitrary entities, such as scent emitter 111; controlled power source 102 which supplies power to an arbitrary electrical device through conventional power plug 103; controlled appliance 101, which in this case is shown as an air filtering device; light source 104; and/or other devices incorporating the SECN control mechanisms, all of which can receive instructions through control messages 524. Directors 122 and 123 are examples of the collective class director (not shown in the figures). Combined configurator/director 151 combines the functions of configurator 141 and director 123 in a single component.

Directors 123 are primarily responsible for delivering requests to controllers. A director can also deliver new behavior modules 801 to controllers and receive reports back about controller operation and about the device(s) it manages. Directors 123 can range from very simple (e.g., fixed-function director 122, which may be a wall-mounted switch that only requests scent emitters to turn on and off) to relatively sophisticated (e.g., flexible director 123 which is a handheld remote control that can control, configure, and interrogate arbitrary controllers 301). Examples of "behaviors" may include responding to specific odors and/or specific odor levels; on/off sequences; and/or alternating scents (i.e., alternating between different air freshening compositions). The alteration of scents may happen within a single scent emitting device that is capable of emitting at least two different air freshening compositions (i.e., having different scents) or from two different scent emitting devices emitting air freshening compositions different each other.

Configurator 141 is typically a graphical software interface run on a commodity computing platform (e.g., desktop PC, laptop, or handheld computer) for designing and configuring behaviors. Such an interface allows a person to use familiar tools and imagery to specify device behavior in a user-friendly manner, and then load the behavior into director 123, which can configure controllers 301 to exhibit that behavior.

Functions of configurator 141 and director 123 are logically distinct: configurator 141 designs-a relatively rare activity-and director 123 controls-something done as a natural part of daily activities. Often, they will be physically distinct: a common implementation would have configurator 141 as software on a desktop or laptop PC, where it would communicate with a director over USB cable interface 142. The functions of director 123 and configurator 141 can also be combined as combined configurator/director 151, for instance in a hand-held computer such as PDA that also includes an interface that can communicate with controllers 301. A set of controllers 301 forms a local area network that may be inherently limited in scope by the type of communication interfaces used by controllers 301. Such networks may be connected to each other, or to the internet, through additional communication interfaces and/or gateway elements that transfer data among multiple such networks, and/or between SECN networks and other networks.

Part of every controlled appliance 101 in the SECN architecture is controller 301. Most types of appliance 101 also incorporate some actual function to be controlled, such as scent emitter 111 which comprises both controller 301 and an air freshening composition (e.g., refill). In the limiting case, appliance 101 may simply control power delivery to some other entity, as in the case of controlled power source 102.

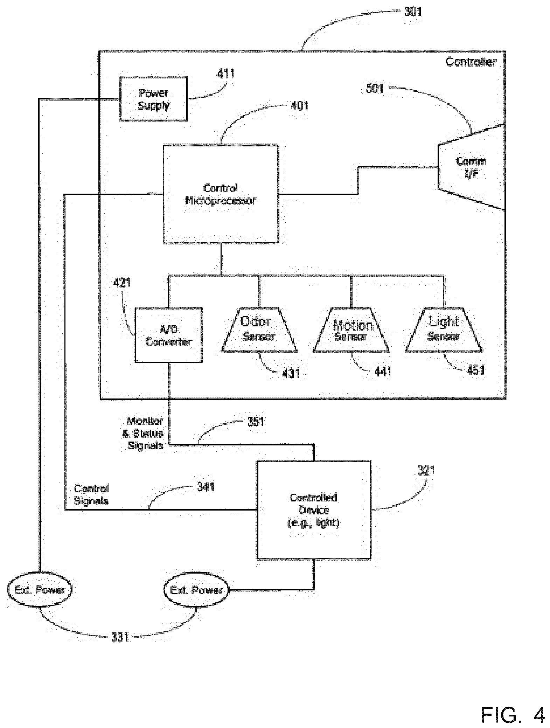

As shown by the example configuration in FIG. 4, controller 301 comprises control microprocessor 401, optionally in combination with some and/or all of power supplies 411, analog-to-digital converters 421, odor sensor 43, motion sensor 441, light sensor 451, communication interfaces 501, and/or other interfaces, sensors, actuators, or mechanisms that enable controller 301 to interact with its environment, of any of which plural instances may be included in controller 301. Although an odor sensor, motion sensor, and light sensor are illustrated in Figure 4, many other sensors can be incorporated / substituted including but not limited to a light sensor, air quality sensor, sound sensor, and the like. A single sensor may have multiple functions (i.e., capable of sensing more than one stimulus), alternatively multiple sensors can be used each with their own specific function. Controller 301 and controlled device 321 are supplied with electrical power from external power supplies 331. Power supplies 331 may be distinct for controller 301 and controlled device 321 as shown in the example, or may be identical. Power supply 411 serves the conventional function of transforming externally-supplied power from power supply 331 into the form (a) required by the internal components of controller 301. A non-limiting example of an odor sensor is described in US 2010/0044453 A1, paragraphs 28 - 29; and US 6,093,308.

Control microprocessor 401 runs software modules called behaviors (section 3 describes a variety of examples) that are loaded into internal memory of controller 401 and that may be subsequently replaced, updated, and/or adjusted. Behavior modules 801 that are running in a controller determine both how it responds to requests and what functions it performs autonomously, for example in response to time-based or sensor input triggers. Controller 301 includes communication interfaces 501 that allow it to communicate with directors 123 and with other controllers 301 (in other appliances 101 such as scent emitters 111).

Controller 301 typically interacts with controlled device 321 through control signals 351, which provide control inputs to the device. Controller 301 typically monitors status and operation of controlled device 321 through status signals 341, which typically are analog voltages or currents that are converted to digital form through analog-to-digital converter(s) 421, although other sensors or interfaces may be used, including digital interfaces of control microprocessor 401. It will be recognized by those skilled in the art that analog-to-digital converter(s) 421 may be integrated with control microprocessor 401, as may other interfaces and sensors.

Although controller 301 can be used in a stand-alone manner, simply controlling power for an arbitrary electrical device, more typically controller 301 is integrated into another electrical device (e.g., lighting device or air filtering device). A scent emitter device 111 is the integration of controller 301, optionally including a sensor, with a refillable air freshening composition.

Controller 301 typically requires a small amount of power to operate, distinct from the power consumed by the device(s) that it controls. It is often desirable for this power supply to be continuously available, even though external power may be completely disconnected from the controlled device. In such cases, controller 301 can incorporate battery power. Power supply 411 is responsible for converting external AC or DC power input, and for managing battery power, to voltage levels more suitable for control microprocessor 401 and other controller components. In one embodiment, the scent emitter is portable and battery operated.

Controller 301 is fundamentally a software-controlled device. Control microprocessor 401 controls and monitors the operation of controlled devices based on the behavior software modules 801 that have been loaded into it, and also performs communication, power management, and device management functions. It will be evident that the function of control microprocessor 401 could be performed by multiple microprocessors, possibly of different types, for example to allow use of simpler and less microprocessors to perform some simpler but time-critical functions and using a more powerful microprocessor for the more complex behaviors. Control microprocessor 401 incorporates processing capabilities, temporary (operational) storage, and non-volatile storage; it will be evident these elements of control microprocessor 401 may be integrated in a single semiconductor component (which typically is the most cost-effective approach) or may be implemented as separate components.

Controller 301 typically incorporates one or more communication interfaces 501 for communicating with directors 123 and controllers 301 in other system elements. The SECN communication protocols can be carried over a wide variety of physical interfaces, including infrared, ultrasonic, radio, power line modulation, light modulation, etc. Communication interface 501 typically supports two-way and symmetric communication, but a one-way communication such as X10 power-line modulation, voice recognition, or simple infrared remote control can be used for simple control functions.

Controller 301, particularly when used for controlling a scent emitting device, typically incorporates one or more sensors 431 for measuring, for example, an odor or an odor level (via an odor sensor). The "odor" may be either a malodor (i.e., generally an unpleasant scent); or absence or low level of freshness (i.e., a perfume scent). These sensors can be used for feedback control of freshness intensity based on external factors such a person's use of an aerosol air freshener or scent cleaning product as well as for compensation for a malodor event (e.g., cooking fish and accompanying unpleasant fish scent or bathroom odors). Multiple odor sensors 431 may be used for different purposes, such as measurement of ambient odor or air quality, measurement of malodor from a room or location within the macro environment, and/or direct measurement of air freshening composition output. Odor sensors 431 may incorporate technology to allow for the measurement specific chemicals in the air. Scent emitters may emit customized air freshening compositions based upon the specific malodor chemical detected in the air. For example, a sulfur based malodor may be treated by the emission of an air freshening composition that works particularly well or is designed to treat sulfur-based malodors. In another embodiment, the odor sensor may be connected remotely to an alarm in the event, for example, smoke or carbon monoxide is detected.

Controller 301 may incorporate one or more sound sensors (e.g., microphones) - not shown. These sensors may be used to enable voice or sound-activated control of the sent emitter device, as an input to be considered in occupancy sensing control, and/or as part of an ultrasonic communication and/or location-mapping function.

Controller 301 may incorporate several voltage-measurement sensors (analog-to-digital converters 421) that allow control microprocessor 401 to monitor relevant aspects of the operation of appliance 101, such as power consumption and/or LED junction voltage drop. Junction voltage drop can provide a measurement of junction temperature, which in turn can be used for feedback control and lifetime monitoring.

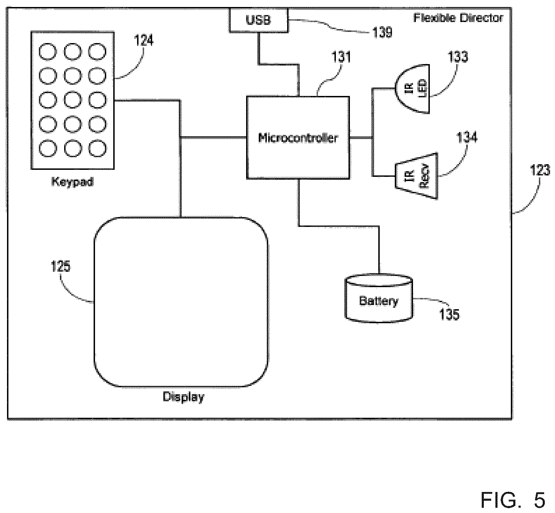

Controller 301 may incorporate one or more infrared or other types of motion sensors 461 (shown in FIG. 5), in order to support control behaviors such as occupancy sensing and response. Such sensors can generate an electrical signal that it is interpreted by the control microprocessor to identify potential motion.

Controller 301 may incorporate one or more video/image sensors (connected similarly to motion sensors 461) that can be used to support behaviors such as occupancy sensing and response. Such sensors can generate pixel image that is processed and interpreted by control microprocessor 401 to identify potential motion. An image sensor could include optics such as a fish-eye lens to allow coverage of the full field visible from the device.

Director 123 is used to send requests to one or more controllers 301, deliver behavior modules 801 to controllers 301, and/or to receive status reports from controllers 301. Director 123, in one embodiment, is similar to a conventional infrared remote control such as might be used with a television set. However, unlike such controls, which only transmit signals and do not receive them, director 123 typically uses a two-way communication protocol to interact with controllers 301 and other directors 123, just as controllers 301 do to interact with each other. This two-way protocol uses acknowledgments and retransmission to allow the director to perform more reliably, and to perform more sophisticated functions, than a conventional remote control.

Typically, director 123 includes a control microprocessor, at least one communication interface (e.g., infrared, ultrasonic), and one or more human operator interfaces (e.g., buttons, knobs, switches). Director 123 may also include a display to allow the operator to view the response to a request and/or to review and/or observe details of a request. The examples described herein are based around two types of director, the simple fixed-function director 122 and the more powerful and sophisticated flexible director 123 shown in Figure 5. It will be evident to one skilled in the art that these distinctions are arbitrary, and that the functions that might be performed by director 123 can be packaged in a virtually limitless variety of packages and configurations (including the combined configuration of configurator/director.

Fixed-function director 122 is very simple, typically used as a "light-switch replacement". A flexible director 123 is shown in FIG. 5. This device is physically similar to a sophisticated handheld remote control: it may include multiple buttons and/or knobs for its operator interface shown as keypad 124. It typically also includes display 125 to allow the operator to see responses send in return to requests by flexible director 123. Its communication interface is typically capable of operating directionally so that the operator can point it at a specific scent emitter 111 to direct requests to that scent emitter alone (which, of course, may forward the request to other scent emitters in a group or groups). Control microprocessor 131 in flexible director 123 typically has sufficient memory both for the director's own software and for storing behavior modules to be delivered to scent emitters 111. Flexible director 123 typically also includes USB interface 139 or other computer-oriented interface to allow it to be updated by configurator 141. Other components in flexible director 123 (infrared transmitter 133, infrared receiver 134, battery 135) serve the same purpose as in fixed-function director 122. An alternative embodiment of flexible director 123 employs a handheld computer equipped with appropriate infrared transmitter 133 and infrared receiver 134 peripherals and appropriate operating software.

Configurator 141 is a software application, running on some hardware platform that is used to design behaviors. It provides a user-focused graphical interface that allows the user to describe the desired behavior of a set of controllers 301 (e.g., those contained in scent emitters 111 and/or sensor(s) 431, 441, 451).

After designing the behaviors with configurator 141, the user then typically transfers the resulting behavior modules 801 into some flexible director 123. Flexible director 123 can then be used to deliver the specified behaviors to some controller 301, which would then, as appropriate, use its communication interface to ensure that the behavior modules 801 are delivered to all controllers 301 that require them.

The SECN architecture explicitly allows the functions of configurator 141 and director 123 to be implemented independently, because that corresponds to a common usage model: an operator could use the powerful graphical interface of configurator 141 running on a personal computer to design or adjust scent emitting behaviors for a space (i.e., macro- or microenvironments).

In some applications, it is more appropriate to combine the functions of director 123 and configurator 141. For example, an operator managing an entire building's freshness profile, or obtaining sensor(s) information for a large area, may use a combined configurator/director 151 unit, which could be portable (such as a iPAD or iPHONE or Tablet PC device) or in a fixed location. In such applications, additional communication gateway components might be employed, for example using standard network, wired, or wireless communication from the operator's computer system to reach a communication gateway component 161 in the areas where targeted controllers 301 are present. Such gateway components could be particularly helpful when an existing building management system (e.g., based on the ZigBee or LONWorks or 6lowpan protocols) is present; alternatively, it is possible to build controllers that incorporate those communication interfaces directly and to use them the communication among SECN components.

Although not shown in the figures, in one embodiment of the invention, the system may comprise at least one sensor that is not integrated with any of the scent emitters (or other appliances) of the system. Valuable information about the environment (e.g., malodor event) may be transmitted by the sensor but where the user does not care for the emission of an air freshening composition. For example, an odor sensor may be placed near a stove where food is being prepared. The odor sensor may sense a malodor emitted from cooking food (e.g., unpleasant fish smell) but the user does not care to have an air freshening composition emitted near where food is being prepared. However, having the sensor in such close proximity to a stove or where food is being prepared provides valuable information (e.g., early warning) about a malodor event to the system. Of course the sensor may even communicate information about the type of malodor and potential quantitative information (e.g., how much malodor). In this embodiment, the non-integrated sensor may comprise a second communications interface configured to communicate with the scent emitters in the network. Alternatively, the sensor has a communication interface capable of transmitting sensor information wirelessly, preferably where the transmission is by way of infrared radiation, ultrasound wave, radio-frequency wave, or combinations thereof.

An air filtering device may be part of the system of the present invention. A non-limiting example of such a device includes those described by US 2009/0038480 A1. In the most basic aspect, the air filtering device is configured to functionally receive a filter (typically a replaceable filter) for removing particles or contaminants from air. The device will also have an air filtering component configured to move air through the air filter. The device may have an air filter functionally attached. The air filter may be replaceable. Typically the air is moved by way of a motorized fan. Generally, the more power or speed of the fan, the greater the air flow through the air filter and thus air filtering. The air filtering device may comprise an air filtering processor. This processor is configured: to have the air filtering component control air flow through the air filter; and to make air filtering decisions based on information received wirelessly, alternatively through the disturbed processing across the system based on information exchanged among the scent emitters (or appliances of the system) through the communication interface. These filtering decisions include turning the air filtering device "on/off' or rate of air flow (e.g., fan speed) or other related functions. In one embodiment, the filtering decision is response to one or more sensors. The sensor may or may not be part of the system. In yet another embodiment, the sensor is configured to sense air quality (e.g., sensing particles or contaminants in the air). In yet still another embodiment, the scent emitter and the air filtering device are integrated into a single device. The filter may also filter bacteria, viruses, and even offending chemical agents and the like. The sensor may be one that detects whether the filter needs to be replaced (e.g., by sensing the quality of the air immediately after flowing through the filter).

### Scent Emitter Behaviors

Because it is a software-controlled device, scent emitter 111 can be programmed to perform a wide variety of functions. A variety of such functions is described below, using scent emitter 111 as the example embodiment. It is understood that activities attributed to scent emitter 111 are in fact carried out by the controller 301 component of scent emitter 111, employing software running on control microprocessor 401 component of controller 301.

Scent emitter 111 may respond to requests (e.g., from director 123) that instruct it to perform specific functions. A typical set of direct requests accepted by scent emitter 111 could include: on/off; scent intensity; scent selection; malodor detection; and the like.

Scent emitter 111 may be part of a group of scent emitters that all are intended to respond similarly. To accomplish this, communication interface 501 can be used to pass on the requests from one scent emitter to others, until all members of the group have been informed of the request. To ensure reliable transfer, positive acknowledgement would typically be part of such a communication protocol.

The simplest application of group control is to have all the scent emitters in a group respond to direct control requests. However, group control assists in providing many of the other behavior functions when multiple scent emitters are involved, as it allows scent emitters to cooperate in exhibiting similar behaviors.

Scent emitter 111 can be manually assigned to groups using director 123. The director can instruct a given scent emitter 111 that it is to belong to a designated group or groups or, alternatively, that it no longer belongs to a designated group or groups.

Preferably, scent emitters 111 can associate into groups autonomously, based on the ability to communicate with each other, using conventional distributed processing algorithms. Since the preferred mechanisms (e.g., infrared, ultrasound) for communication interface 501 are generally localized to a single open (that is, they are blocked by walls and doors), the scent emitters in such an area can identify themselves to each other and form a group based on reachability.

Scent emitter 111 can adjust the intensity or strength of its communication transmissions, and/or the sensitivity of its communication receiver, to dynamically adjust the distance over which group detection takes place. Each scent emitter 111 can belong to multiple groups, allowing requests received by a particular scent emitter to have different scope depending on the group or groups to which they are addressed.

Scent emitters 111 can keep track of the date and time of day and exhibit behaviors triggered at specific times. For example, an scent emitter (or group of scent emitters) can be requested to turn on during waking hours and off at night. Scent emitters 111 can be informed of the current date and time by director 123. Director 123 can similarly be informed of the time by configurator 141, which can obtain accurate highly accurate time from network time references, for example by using the Network Time Protocol (NTP) or the Simple Network Time Protocol (SNTP). Alternatively, the time can be programmed into the scent emitter by the user.

Scent emitter 111 can record the requests it has been given and repeat them at a later time, for example allowing it to learn desired on/off times on one day and repeating them on subsequent days. Such learning could, for example, be adjusted by knowledge of specific days, weekends, and holidays, allowing repetition of desired behavior on appropriate days, for example
Mimicking a week's use of air freshening.

Scent emitter 111 can incorporate sensors to detect presence of human occupants, enabling it to reduce the consumption of energy consumption and/or air freshening composition by providing freshness only when needed. If the sensor(s) detect(s) no indication of occupancy for an extended period, the scent output can be turned off or decreased in intensity. For example, motion sensor 461 can be a conventional long-wave infrared motion sensor can be used for detecting motion of warm bodies. Control microprocessor 401 can monitor and integrate the output of motion sensor 461 over a relatively long period to avoid accidentally turning off lights while someone is present. The motion sensor 461 can be a video image sensor. Sound sensor 451 (e.g., a microphone) can also be used for occupancy detection. Typically, if multiple sensors are present (e.g., sound sensor 451 and motion sensor 461), the scent emitter device 111 would combine signals and signal-derived conclusions from the different sensors to provide a more reliable overall detection of occupancy.

Scent emitter 111 can incorporate sound sensor 451 (e.g., a microphone) and voice recognition software in control microprocessor 401 to allow it to respond to voice requests. Limited-vocabulary voice recognition software is widely available commercially, and is used in applications such as interactive toys and hands-free telephones.

Scent emitter 111 can measure and/or calculate a variety of characteristics about its operation including air freshening composition consumption, refill life remaining, power consumption, malodor events, total operating hours. Such status information can be accumulated by control microprocessor 401 and reported back to configurator 141 or other destination through director 123 or gateway 161. Such status information can also be communicated directly to a director 123. Such reporting is to allow such information to be tracked and predicted. For example, air freshening composition refills can be ordered/delivered (even automatically) - before the refill is empty.

In combination with location identification and awareness, a set of scent emitters 111 can cooperate to provide a balanced adjustment of scent intensities and/or malodor control in response to a request directed at a single scent emitter or sensor or combination scent emitter/sensor. Similarly, autonomous control behaviors may, through communication among scent emitters, provide a pre-programmed experience.

Parameters governing behavior response may be selected from a set of template behaviors, or may be explicitly programmed, through the interface provided by configurator 141 and director 123. For example, in an occupancy response behavior, parameters could govern the length of time required without an indication of occupancy after which a scent emitter 111 would conclude that there are no humans present. Similarly, parameters could govern the amount and/or type of signal required from motion detection sensor 461 used for occupancy detection that should considered as a positive indication of occupancy.

### Communication

Every controlled appliance 101 (e.g., scent emitter 111, controlled power source 102; that is, any element that incorporates controller 301), as well as every director 123, gateway 161, and combined configurator/director 151 incorporates at least one communication interface 501. Typically, communication interface 501 is bi-directional and can both send and receive messages (not necessarily simultaneously); however, in some cases (e.g., fixed function director 122), only a one-way interface is required.

Controllers 301 and directors 123 communicate with each other to receive and acknowledge requests, to deliver reports, and to forward requests throughout a constellation of controlled appliances 101. Controllers 301 communicate with each other to forward and deliver messages of all types and to ensure, through use of acknowledgments, that messages are delivered to all controllers that are intended to receive them.

Communication can be viewed as three logical layers: the physical layer used to transmit bits from one component to another; the network layer used to manage communication among the components; and the application layer, used to coordinate the activities of multiple components. The physical layer is implemented in part by communication interface 501, which is a hardware component that sends and receives data. Software running in control microprocessor 401 may implement part of the physical communication layer as well, performing modulation and demodulation to transform between raw electromagnetic signals used by communication interface 501 and digital data comprising messages. Common physical communication techniques are infrared and ultrasound, although radio, hardwired, power line modulation, and/or other techniques can also be used if appropriate. The network layer provides for transport of data between senders and recipients, and also may provide either a direct or emulated multicast capability. In the SECN architecture, the data transfers are almost always short, so communication can be optimized for such traffic. Lastly, the application layer manages message exchanges between software modules 801 running in different controllers 301, enabling them to coordinate their activities and providing a reliable transmission service to store and forward messages.

Gateway 161 elements can be used to integrate the SECN communication mechanisms with other networks, allowing information (such as requests and reports) to be delivered over the Internet and/or private networks. Conventional network security mechanisms (e.g., authentication, encryption, firewalls) can be used to protect an SECN network from unauthorized use or access. An SECN network can also be used as transport for information from other networks, e.g., by mechanisms such as IP tunneling. Integration with the Internet and private IP networks allows SECN elements to be controlled and interrogated from arbitrary locations, facilitating remote control and building management.

### Controller Software

Software running in control microprocessor 401 is responsible for implementing all the control, communication, monitoring, and behavior functions performed by controller 301. Because cost is typically an important consideration for the implementation technology of controller 301, the software is typically optimized to minimize resource requirements and runtime cost. Activities typically occur within control microprocessor 401 on plural distinct timescales. In the example embodiment of scent emitter 111, those timescales include high-rate, medium-rate, and low-rate activities. Mid-rate activities are timer-driven and typically occur at a "housekeeping interval" at about 480 times per second. They activities include management of the high-resolution software clock, adjusting power control parameters, scheduling high-rate activities, and monitoring sensor inputs. High-rate activities are both event-driven and timer-driven, and can occur at rates up to 25,000 times/second. High-rate activities include LED power control setting, IR transmitter bit generation, and IR receiver bit recognition. Low-rate activities occur at much lower time scales than the housekeeping interval: typically seconds or minutes. These include various types of status monitoring and communication.

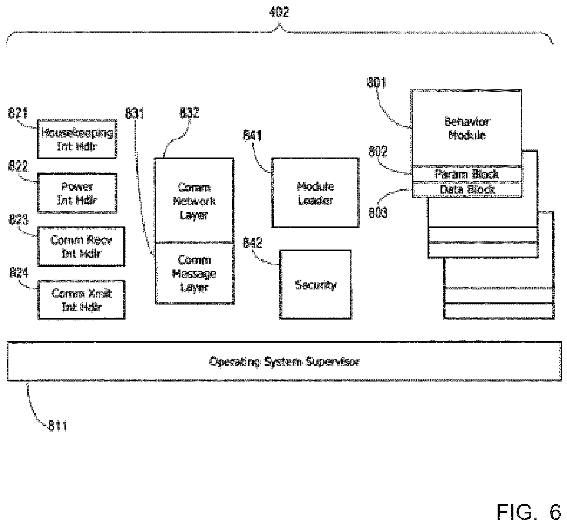

In the example embodiment of scent emitter 111, control microprocessor 401 in controlled 301 would typically run control software 402 consisting of the following elements, as shown in FIG. 6: Operating system supervisor 811; Housekeeping interrupt handler 821; Power control interrupt handler 822; Communication receive interrupt handler 823; Communication transmit interrupt handler 824; Communication message layer 831; Communication network layer 832; Module manager 841; Security library 842; Plural behavior modules 801; Plural external parameter blocks 802; and Plural internal data blocks 803.

Operating system supervisor 811 is a tiny real-time operating system kernel that supplies services for task dispatching, inter-task communication, and memory management.

Housekeeping interrupt handler 821 is a timer-driven interrupt-handling module that performs the mid-rate housekeeping tasks. It keeps track of real time and ensures that high-rate and low-rate activities are scheduled appropriately.

Communication receive interrupt handler 823 processes interrupts from communication interface 751, which typically indicate receipt of one or more bits of a network message, and which are deposited into a message input buffer, but may also be noise that can be recognized and rejected by communication receive interrupt handler 823. Communication receive interrupt handler 823 is typically invoked only in response to external events and is not timer-driven.

Communication transmit interrupt handler 824 is a timer-driven module that controls the output (transmit) aspect of communication interface.

Communication message layer 831 is responsible for formatting and addressing network messages, managing input and output buffers. It sets up the parameters and buffers that drive communication interrupt handlers 823 and 824, and performs other typical tasks associated with the Open Systems Interconnection (OSI) layered network model.

Communication network layer 832 is responsible for managing application communication in the overall network of controllers 301, ensuring that messages are delivered to required recipients, processing acknowledgments, and performing other typical communication tasks associated with the Network and Session layers of the OSI network model, managing input and output buffers. It sets up the parameters and buffers that drive communication interrupt handlers 823 and 824.

Module manager 841 loads and unloads behavior modules 801 and associated data blocks 802 and 803. It is responsible for validating modules, managing memory, maintaining associations between modules and data blocks, associating modules with network message types delivered by network layer 832, managing dependencies among modules, assembling modules from fragments during module download and delivery, and other tasks associated with behavior modules 801. Module manager 841 is also responsible for managing dynamic updates to other software components of control software 402, and for updating and accessing external parameter data blocks 802 and internal data blocks 803.

Security library 842 provides cryptographic functions for authentication, encryption, decryption, key management, and other purposes. Cryptographic functions can used by module manager 841 to validate modules, by communication message layer 831 or network layer 832 to protect network messages, and/or for any purpose required in some behavior module(s) 801.

Behavior module(s) 801 are executable modules that can be loaded in arbitrary combinations into control microprocessor 402. They can implement above-described behaviors and/or arbitrary other functions. A behavior module 801 typically has an associated external parameter data block 802 that specifies parameters to control the behavior, and may also have an associated internal data block 803 that maintains, internally to control microprocessor 401, non-volatile storage for information relevant to that behavior module 801. Behavior modules 801 can have metadata that identifies dependencies and allows for version management.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An air freshening system comprising:
(a) a plurality of scent emitters displaced relative to each other in the freshening system, wherein each of the plurality of scent emitters is configured to emit an air freshening composition;
(b) at least one sensor responsive to a stimulus the sensor is capable of sensing;
(c) a communication interface configured to communicate with the plurality of scent emitters;
(d) a director; and
(e) wherein each of the plurality of scent emitters comprises an emission processor and the director is configured to deliver a user's requests to the emission processors, wherein each emission processor is:
(i) responsive to the sensor;
(ii) configured to controlling scent emission from the respective scent emitter; and
(iii) configured to make scent emission decisions through distributed processing across the system based on information exchange among the scent emitters through the communication interface; and
(iv) configured to learn behavior desired by the user by responding to the user's requests and modifying its behavior in response to the user's requests.

2. The system of claim 1, further comprising a radio-frequency gateway allowing scent emitters of the system to communicate with each other.

3. The system of claim 2, wherein the sensor is selected from an odor sensor or a motion sensor.

4. The system of claim 3, wherein the system comprises a plurality of sensors wherein at least one sensor is a malodor sensor and another sensor is a motion sensor.

5. The system of claim 3, wherein the at least one sensor is integral to at least one scent emitter of the system.

6. The system of any preceding claim, wherein the scent emitter further comprises an energizable dispenser component configured to dispense an air freshening composition from a refill vial.

7. The system of any preceding claim wherein the scent emitter is pluggable into a wall socket.

8. The system of any preceding claim, wherein the emission processor comprises using one or more of the following: a clock to control the timing of scent emission decisions across the network; and learning reactions to stimuli according to a training set.

9. The system of any preceding claim, further comprising an air filtering device configured to filter air, wherein the air filter device comprises:
(a) an air filtering component configured to move air through an air filter;
(b) the air filter functionally attached to the air filter device;
(c) an air filtering processor, wherein the air filtering processor is configured: to have the air filtering component control air flow through the air filter; and to make air filtering decisions based on information received through the distributed processing across the system based on information exchanged among the scent emitters through the communication interface.

10. The system of any preceding claim, wherein the filtering process of the air filter device is further configured to be responsive to the sensor.

11. The system of any preceding claim, wherein the sensor further comprises an air quality sensor.

12. The system of any preceding claim, wherein the communication interface is configured to send or receive information by infrared radiation, ultrasound wave, radio-frequency wave, or combinations thereof.

13. The system of any preceding claim, wherein the scent emitter further comprises a radio-frequency gateway.

## Patentansprüche

1. Luftauffrischungssystem, das umfasst:
(a) eine Vielzahl von Duftspendern, die in dem Auffrischungssystem in Bezug zueinander versetzt sind, wobei jeder der Vielzahl von Duftspendern so konfiguriert ist, dass er eine Luftauffrischungszusammensetzung abgibt;
(b) mindestens einen Sensor, der auf einen Reiz anspricht, den der Sensor erfassen kann;
(c) eine Kommunikationsschnittstelle zum Kommunizieren mit der Vielzahl von Duftspendern;
(d) einen Sollwertgeber; und
(e) wobei jeder der Vielzahl von Duftspendern einen Abgabeprozessor umfasst und der Sollwertgeber dafür konfiguriert ist, Benutzeranforderungen an die Abgabeprozessoren zu liefern, wobei jeder Abgabeprozessor:
(i) auf den Sensor reagiert;
(ii) dafür konfiguriert ist, die Duftabgabe von dem jeweiligen Duftspender zu steuern; und
(iii) dafür konfiguriert ist, Duftabgabeentscheidungen durch eine verteilte Verarbeitung über das System hinweg basierend auf einem Informationsaustausch zwischen den Duftspendern durch die Kommunikationsschnittstelle zu treffen; und
(iv) dafür konfiguriert ist, das vom Benutzer gewünschte Verhalten zu erlernen, indem es auf die Benutzeranforderungen reagiert und sein Verhalten als Reaktion auf die Benutzeranforderungen modifiziert.

2. System nach Anspruch 1, das ferner ein Funkfrequenz-Gateway umfasst, das es Duftspendern des Systems erlaubt, miteinander zu kommunizieren.

3. System nach Anspruch 2, wobei der Sensor aus einem Geruchssensor oder einem Bewegungssensor ausgewählt ist.

4. System nach Anspruch 3, wobei das System eine Vielzahl von Sensoren umfasst, wobei mindestens ein Sensor ein Sensor für üble Gerüche ist und ein anderer Sensor ein Bewegungssensor ist.

5. System nach Anspruch 3, wobei der mindestens eine Sensor integral mit mindestens einem Duftspender des Systems ist.

6. System nach einem der vorstehenden Ansprüche, wobei der Duftspender ferner eine strombeaufschlagbare Spenderkomponente umfasst, die dafür konfiguriert ist, eine Luftauffrischungszusammensetzung aus einem Nachfüllfläschchen abzugeben.

7. System nach einem der vorstehenden Ansprüche, wobei der Duftspender in eine Wandsteckdose gesteckt werden kann.

8. System nach einem der vorstehenden Ansprüche, wobei der Abgabeprozessor das Verwenden eines oder mehrerer der Folgenden umfasst: einen Takt zum Steuern der zeitlichen Planung von Duftabgabeentscheidungen über das Netzwerk hinweg; und Erlernen von Reaktionen auf Reize gemäß einem Übungsset.

9. System nach einem der vorstehenden Ansprüche, das ferner eine Luftfiltervorrichtung umfasst, die dafür konfiguriert ist, Luft zu filtern, wobei die Luftfiltervorrichtung umfasst:
(a) eine Luftfilterkomponente, die dafür konfiguriert ist, Luft durch einen Luftfilter zu bewegen;
(b) den Luftfilter, der funktionell an der Luftfiltervorrichtung angebracht ist;
(c) einen Luftfilterprozessor, wobei der Luftfilterprozessor dafür konfiguriert ist, zu bewirken, dass die Luftfilterkomponente die Luftströmung durch den Luftfilter steuert; und dafür, Luftfilterungsentscheidungen basierend auf Informationen zu treffen, die durch die verteilte Verarbeitung über das System hinweg empfangen werden, basierend auf Informationen, die zwischen den Duftspendern durch die Kommunikationsschnittstelle ausgetauscht werden.

10. System nach einem der vorstehenden Ansprüche, wobei der Filterprozess der Luftfiltervorrichtung ferner so konfiguriert ist, dass er auf den Sensor reagiert.

11. System nach einem der vorstehenden Ansprüche, wobei der Sensor ferner einen Luftqualitätssensor umfasst.

12. System nach einem der vorstehenden Ansprüche, wobei die Kommunikationsschnittstelle dafür konfiguriert ist, Informationen durch Infrarotstrahlung, Ultraschallwellen, Funkfrequenzwellen oder Kombinationen davon zu senden oder zu empfangen.

13. System nach einem der vorstehenden Ansprüche, wobei der Duftspender ferner ein Funkfrequenz-Gateway umfasst.

## Revendications

1. Système désodorisant d'ambiance comprenant :
(a) une pluralité d'émetteurs de senteur déplacés les uns par rapport aux autres dans le système désodorisant, dans lequel chacun parmi la pluralité d'émetteurs de senteur est configuré pour émettre une composition de désodorisant d'ambiance ;
(b) au moins un capteur qui réagit à une stimulation que le capteur est susceptible de détecter ;
(c) une interface de communication configurée pour communiquer avec la pluralité d'émetteurs de senteur ;
(d) un contrôleur ; et
(e) dans lequel chacun parmi la pluralité d'émetteurs de senteur comprend un processeur d'émission et le contrôleur est configuré pour délivrer des demandes d'un utilisateur aux processeurs d'émission, dans lequel chaque processeur d'émission :
(i) réagit au capteur ;
(ii) est configuré pour commander une émission de senteur à partir de l'émetteur de senteur respectif ; et
(iii) est configuré pour prendre des décisions d'émission de senteur par l'intermédiaire d'un traitement réparti à travers le système sur la base d'un échange d'informations parmi les émetteurs de senteur par l'intermédiaire de l'interface de communication ; et
(iv) est configuré pour apprendre un comportement souhaité par l'utilisateur en répondant à des demandes de l'utilisateur et en modifiant son comportement en réponse aux demandes de l'utilisateur.

2. Système selon la revendication 1, comprenant en outre une passerelle radiofréquence permettant aux émetteurs de senteur du système de communiquer les uns avec les autres.

3. Système selon la revendication 2, dans lequel le capteur est choisi parmi un capteur d'odeurs ou un capteur de mouvement.

4. Système selon la revendication 3, dans lequel le système comprend une pluralité de capteurs dans lequel au moins un capteur est un capteur de mauvaises odeurs et un autre capteur est un capteur de mouvement.

5. Système selon la revendication 3, dans lequel l'au moins un capteur est solidaire d'au moins un émetteur de senteur du système.

6. Système selon une quelconque revendication précédente, dans lequel l'émetteur de senteur comprend en outre un composant distributeur excitable configuré pour distribuer une composition de désodorisant d'ambiance à partir d'un flacon de recharge.

7. Système selon une quelconque revendication précédente, dans lequel l'émetteur de senteur peut être branché dans une prise murale.

8. Système selon une quelconque revendication précédente, dans lequel le processeur d'émission comprend l'utilisation d'un ou plusieurs parmi les suivants : une horloge pour commander la temporisation de décisions d'émission de senteur à travers le réseau ; et des réactions d'apprentissage à des stimulations selon un ensemble d'apprentissage.

9. Système selon une quelconque revendication précédente, comprenant en outre un dispositif de filtrage d'air configuré pour filtrer l'air, dans lequel le dispositif de filtre à air comprend :
(a) un composant de filtrage d'air configuré pour déplacer de l'air à travers un filtre à air ;
(b) le filtre à air fonctionnellement fixé au dispositif de filtre à air ;
(c) un processeur de filtrage d'air, dans lequel le processeur de filtrage d'air est configuré : pour avoir le composant de filtrage d'air qui commande le flux d'air à travers le filtre à air ; et pour prendre des décisions de filtrage d'air sur la base d'informations reçues par l'intermédiaire du traitement réparti à travers le système sur la base d'informations échangées parmi les émetteurs de senteur par l'intermédiaire de l'interface de communication.

10. Système selon une quelconque revendication précédente, dans lequel le processus de filtrage du dispositif de filtre à air est en outre configuré pour réagir au capteur.

11. Système selon une quelconque revendication précédente, dans lequel le capteur comprend en outre un capteur de qualité d'air.

12. Système selon une quelconque revendication précédente, dans lequel l'interface de communication est configurée pour envoyer ou recevoir des informations par rayonnement infrarouge, onde ultrasonore, onde radiofréquence, ou des combinaisons de ceux-ci.

13. Système selon une quelconque revendication précédente, dans lequel l'émetteur de senteur comprend en outre une passerelle radiofréquence.
